Europäisches Patentamt

(19) European Patent Office

Office européen des brevets

(11) EP 0 812 594 A1

## (12) EUROPEAN PATENT APPLICATION

(43) Date of publication:
17.12.1997 Bulletin 1997/51

(21) Application number: 96109483.6

(22) Date of filing: 13.06.1996

(51) Int. Cl.$^6$: **A61K 49/00**, A61K 31/00

(84) Designated Contracting States:
DE

(71) Applicant:
BOEHRINGER INGELHEIM INTERNATIONAL GmbH
55218 Ingelheim am Rhein (DE)

(72) Inventors:
- Nasmyth, Kim
  A-1010 Wien (AT)
- Piatti, Simonetta
  I-20133 Milano (IT)
- Diffley, John
  St. Albans, Herts AL3 5JH (GB)

(54) **Compounds that interfere with DNA replication in rapidly proliferating cells for use in cancer therapy and methods for screening for such compounds**

(57) Compounds which interfere with the function of the cdc6 protein to form or maintain pre-replication complexes in an animal cell without impairing the cell's ability to activate cyclin dependant kinases that promote S phase and/or M phase for use in cancer therapy due to their ability to kill tumour cells by inhibiting DNA replication. Method for screening for such compounds.

There is now considerable evidence that chromosome duplication is triggered by the activation in late G1 of particular cyclin dependent kinases, known as S phase promoting Cdks.

It was an object of the present invention to further investigate the mechanisms involved in DNA replication and, utilizing the obtained results, to provide a novel concept for cancer therapy which, as opposed to conventional chemotherapy, does not damage DNA *per se* but interferes with DNA replication.

EP 0 812 594 A1

## Description

The present invention relates in general to the field of cancer therapy.

There is now considerable evidence that chromosome duplication is triggered by the activation in late G1 of particular cyclin dependent kinases, known as S phase promoting Cdks.

It was an object of the present invention to further investigate the mechanisms involved in DNA replication and, utilizing the obtained results, to provide a novel concept for cancer therapy which, as opposed to conventional chemotherapy, does not damage DNA *per se* but interferes with DNA replication.

In the budding yeast *Saccharomyces cerevisiae*, entry into S phase depends on Cdks formed by the association of one of six unstable B-type cyclins, Clb1-6, with a single kinase subunit, Cdk1/Cdc28 (Schwob et al., 1994). Cyclin B/Cdk1 kinases are also required for the assembly of a mitotic spindle and for the onset of anaphase in yeast (Surana et al., 1991; Fitch et al., 1992; Schwob and Nasmyth, 1993).

All six cyclin B/Cdk1 kinases are inactivated as cells complete anaphase (Amon et al., 1994; Irniger et al., 1995). Their re-activation during the subsequent G1 period involves periodic transcription of cyclin genes (Koch and Nasmyth, 1994), cessation of cyclin B proteolysis (Amon et al., 1994), and, most important, destruction of p40$^{SIC1}$ which otherwise inhibits cyclin B/Cdk1 kinases through the formation of ternary p40$^{SIC1}$/cyclinB/Cdk1 complexes (Mendenhall, 1993; Schwob et al., 1994). Inactivation of all six B-type cyclins or mutations in genes like *CDC4* and *CDC34* , which are needed for p40$^{SIC1}$ proteolysis, cause cells to arrest in G1, whereas inactivation of the *SIC1* gene modestly advances S phase (Schwob et al., 1994). The equivalent S phase promoting Cdks in metazoa appear to be complexes between an S phase specific kinase subunit Cdk2 and a similar set of cyclins, known as E-type cyclins.

It is thought that Clb5/ and Clb6/Cdk1 kinases normally trigger S phase in yeast (Epstein and Cross, 1992; Schwob and Nasmyth, 1993); these are the first B-type cyclins to appear due to transcriptional controls that cause *CLB5* and *CLB6* mRNAs to accumulate in late G1. *CLB3* and *CLB4* are transcribed in S and G2 phases, while *CLB1* and *CLB2* are transcribed in G2 and M phases (Koch and Nasmyth, 1994). Deletion of both *CLB5* and *CLB6* is not lethal but causes S phase to be delayed by about 30 minutes (Schwob and Nasmyth, 1993), i.e. until the later Clbs have had time to accumulate.

It is now clear that one or another of the six S phase promoting cyclinB/Cdk1 kinases are active from the time of p40$^{SIC1}$ destruction in late G1 to the activation of cyclin B proteolysis during anaphase and yet origins fire once and only once during this wide window of the yeast cell cycle. It is possible that S phase promoting Cdks trigger chromosome duplication in G1 but not in G2 cells because the appropriate substrates for S phase promoting Cdks might only exist in G1 cells? Proteins that bind to origins of DNA replication might be the key substrates for Cdks and their availability might vary during the cell cycle. A large multisubunit complex called the Origin Recognition Complex (ORC) binds to and is required for the activity of yeast origins (Bell and Stillman, 1992; Micklem et al., 1993; Liang et al., 1995; Fox et al., 1995). ORC is bound to origins at all stages of the cell cycle (Diffley and Cocker, 1992), but the DNaseI sensitivity of the neighbouring chromatin changes during the cycle (Diffley et al., 1994). Sequences adjacent to ORC are protected from DNaseI during G1 but not during G2 or M phase. The pre-replicative "protected" state of origins arises at the end of mitosis, following the inactivation of Clb/Cdk1 kinases and lasts until the beginning or middle of S phase (Diffley et al., 1994).

The formation and maintainance of the pre-replicative state of origins depends on the synthesis of an unstable protein encoded by the *CDC6* gene (Cocker et al., 1996), which is essential for the initiation of DNA replication (Hartwell, 1976; Bueno and Russell, 1992; Piatti et al., 1995). Defects in the replication of minichromosomes in *cdc6* mutants can be alleviated by adding extra origins, suggesting that Cdc6p (CdC6 protein) acts at origins (Hogan and Koshland, 1992). *Cdc6* transcription occurs in two bursts during the cycle: at the end of mitosis, coinciding with the switch of origins from post-replicative to pre-replicative state, and in late G1 (Zwerschke et al., 1994; Piatti et al., 1995). Cells that exit from mitosis without *de novo* Cdc6 protein synthesis fail both to establish the pre-replicative state at their origins (Cocker et al., 1996) and to initiate DNA replication, despite activating S phase promoting Cdks on schedule; such cells proceed with the alignment of unreplicated chromosomes on mitotic spindles and finally undergo a "reductional" anaphase (Piatti et al., 1995).

These data suggest that Cdc6p synthesis is required for the assembly of protein complexes adjacent to ORC (pre-replicative complexes or pre-RCs). The formation of such pre-RCs at the end of mitosis or in late G1 (when there is a second burst of Cdc6 synthesis) might be the first of two steps needed for the initiation of DNA replication; the second step being the activation of cyclin B/Cdk1 kinases. According to this notion, the presence of pre-RCs in G1 but not in G2 cells could explain why S phase promoting Cdks trigger only the former to enter S phase. Thus, the disassembly of pre-RCs upon origin firing and their failure to re-form during S, G2 and M phases could have an important role in preventing re-replication during the cell cycle.

To address how yeast cells restrict the formation of pre-RCs to a short period following anaphase, it was investigated when during the cell cycle Cdc6 protein is capable of forming pre-RCs and promoting DNA replication. It could be shown that Cdc6p is capable of inducing DNA replication only when synthesised during a narrow window of the cell cycle, between the end of anaphase, when B-type cyclins are destroyed, and a point that roughly coincides with the beginning of the subsequent S phase, when Clb5-6/Cdk1 kinases are reactivated. There exists, therefore, a "point of no return" in late G1, after which Cdc6 protein loses the ability to promote DNA replication, presumably because it can no longer form pre-RCs. The data obtained in the experiments of the invention are consistent with the proposal that assembly of pre-RCs is inhibited by the same set of cyclin B/Cdk1 kinases that trigger DNA replication (Dahmann et al., 1995).

In the experiments of the present invention the following results have been obtained:

1) Cdc6p synthesis is able to promote formation of pre-RCs in α factor but not in nocodazole arrested cells

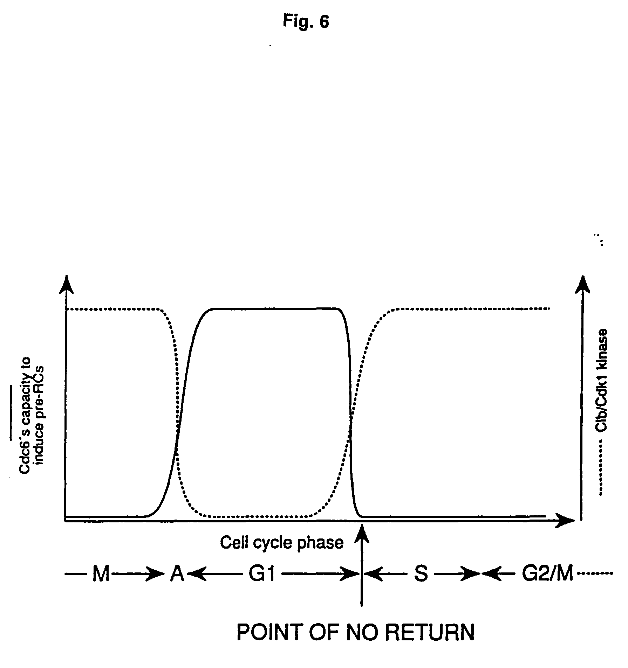

It was shown that *de novo* synthesis of Cdc6p, either as cells exit from mitosis or subsequently in late G1, is necessary for DNA replication in budding yeast but not for progress through other aspects of the cell cycle. Cells that fail to synthesise Cdc6 during G1 proceed with the formation of mitotic spindles and segregate their unreplicated chromosomes at random to mother and daughter cells (Piatti et al., 1995). Inactivation of mitotic Clb/Cdk1 kinases (by elevating the temperature of a strain lacking *CLB1*, *3*, and *4* and carrying a ts allele of *CLB2*), which are required for assembly of mitotic spindles, prevented the abnormal nuclear division of cells deprived of Cdc6 but not, surprisingly, their rapid death; that is, they lost the ability to form colonies when Cdc6 and Clb2 functions were restored (see Fig. 6 in Piatti et al., 1995). This experiment was repeated and the DNA content of cells measured by FACS analysis during the abortive recovery period. It was found that cells were unable to replicate their chromosomes even when Cdc6 synthesis was restored half an hour before shift to the permissive temperature, which restores Clb2 function (data not shown).

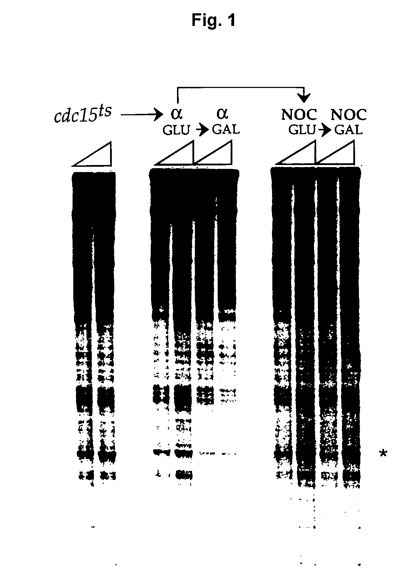

These data could be explained if Cdc6 synthesis were capable of promoting DNA replication only during certain stages of the cell cycle; cells deprived of Clb1-4 might arrest at a stage of the cell cycle during which Cdc6 cannot promote the formation of pre-replication complexes (pre-RCs). To investigate this further, the formation of pre-RCs in response to *de novo* Cdc6 synthesis in cells arrested in a "G1" like state by pheromone was compared with that in cells arrested in a "G2" or "M" phase like state by nocodazole. Due to the lack of Cdc6 synthesis prior to arresting the cells, these two states do not correspond to conventional G1 or G2/M states. G1 cells normally possess pre-RCs at replication origins and nocodazole arrested cells normally have replicated chromosomes. To perform the experiment, a strain was used whose only functional Cdc6 protein was fused to ubiquitin and was expressed from the *GAL1-10* promoter (*GAL-ubiCDC6*). The strain also contained a *cdc15* mutation that caused the arrest of cells in late anaphase by incubation at 37°C. Upon shift down to 25°C in the presence of α factor but in the absence of galactose, cells entered the next G1 phase synchronously without producing Cdc6 protein. This produced a culture of unbudded cells lacking pre-RCs at the 2 μm origin (Fig. 1). The culture was then split and one half incubated for 90 min. in medium still lacking galactose but containing nocodazole, which caused cells to arrest with buds and high levels of Clb/Cdk1 kinase but with unreplicated chromosomes lacking pre-RCs. Re-activation of Cdc6 synthesis by addition of galactose induced formation of pre-RCs (as measured by the protection from DNase I DNA sequences adjacent to those bound by ORC) in the α factor arrested culture but not in the nocodazole one. The failure to detect pre-RCs in nocodazole arrested cells (Diffley et al., 1994) is not, therefore, due to DNA replication itself. These data suggest that *de novo* Cdc6 synthesis cannot induce the formation pre-RCs in cells whose state resembles that of a G2 or M phase cell, even when a prior lack of Cdc6 synthesis has prevented the replication of their chromosomes.

2) Delayed Cdc6 synthesis cannot promote DNA replication

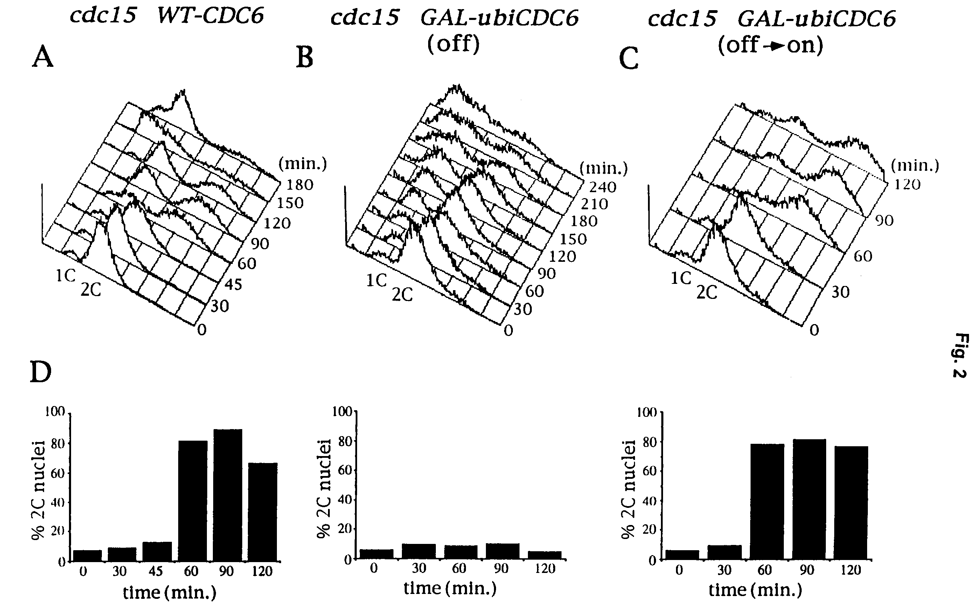

The next experiment was designed to measure more precisely the cell cycle dependence of Cdc6's capacity to promote DNA replication. Synchronised populations of G1 cells were generated that lack Cdc6 protein and turned on Cdc6 synthesis at will during the subsequent progression of these cells through the cell cycle. Cells were synchronised in late anaphase using a ts *cdc15* mutation. Fig. 2A shows cellular DNA contents measured by FACS analysis in the period following release from a *cdc15* arrest in cells containing an intact *CDC6* gene. Cells start with a 2C DNA content (due to 1C chromosome clusters/nuclei at each end of the budded cell) and most, if not all, cells replicate DNA before they complete cell division. A transient accumulation of cells with a 1C DNA content could not be seen (as would have been expected if cells divided before replicating), but instead a transient accumulation of cells with a 4C DNA content (i.e. cells with two 2C nuclei). To use the FACS profiles to estimate the DNA contents of nuclei during this period, the fraction of cells at each time point with single or double chromosome clusters/nuclei was also measured; i.e. uninucleate (U) and binucleate (B) cells. Assuming that all 4C cells are binucleates and that all 1C cells are uninucleate cells that have not yet undergone a reductional anaphase (see later), then the percentage of nuclei that have replicated (%2C) during the first cycle following release can be calculated using the formula: $\%2C = 100.(2.C_4 + U - C_1)/(U + 2.B)$, where $C_1$, $C_2$, and $C_4$ are the fraction of cells at each time point with 1C, 2C, or 4C DNA contents (see Materials and Methods). Application of this formula shows that most nuclei have a 1C DNA content during the *cdc15* arrest and duplicate their DNA within 60 min. following release.

This experiment was repeated with a *cdc15 GAL-*

*ubiCDC6* strain. First cells were synchronised in late anaphase by shifting cells to the restrictive temperature, Cdc6 synthesis was then repressed by removing galactose from the medium, and 30 min. later cells were released from their mitotic arrest (still in the absence of galactose) by lowering the temperature. Under these circumstances, cells exited from their late anaphase arrest in the absence of *de novo* Cdc6 synthesis and, as a consequence, they failed to enter S phase; nuclei retained a 1C DNA content for at least 120 minutes following release (Fig. 2B) but they subsequently (between 210 and 240 min.) underwent a reductional anaphase to produce nuclei with DNA contents less than 1C. Re-addition of galactose to the medium when cells were returned to 25°C allowed 85% of the nuclei to replicate with kinetics similar to *CDC6+* cells, which demonstrates that *de novo* synthesis of Cdc6 from the *GAL* promoter at the time of *cdc15* release is sufficient to drive S phase (Fig. 2C). These data show that formation of pre-RCs, which does not occur in the absence of Cdc6 synthesis during a *cdc15* release (Cocker et al., 1996), is essential for entry into S phase.

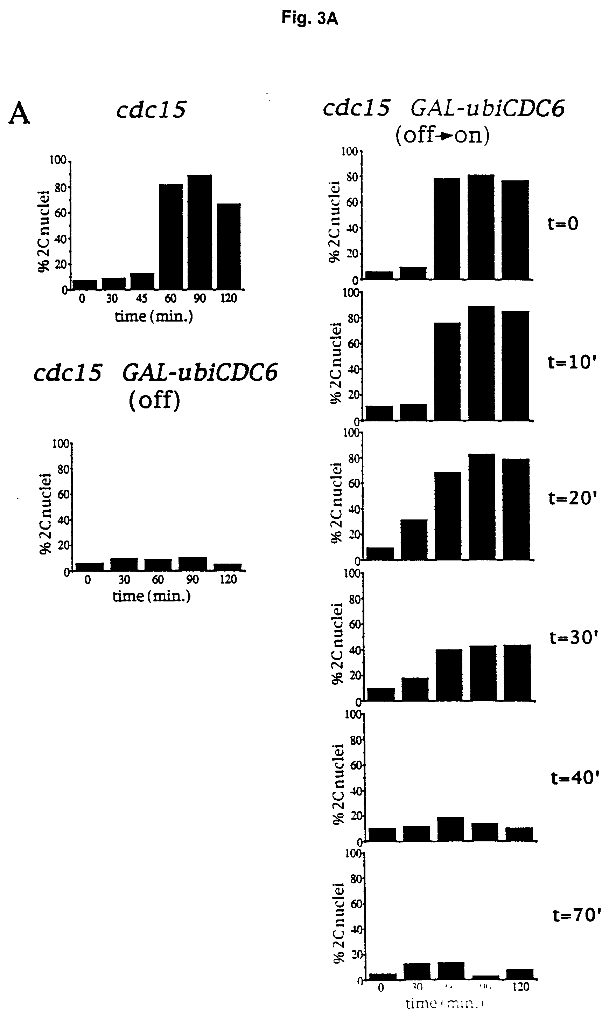

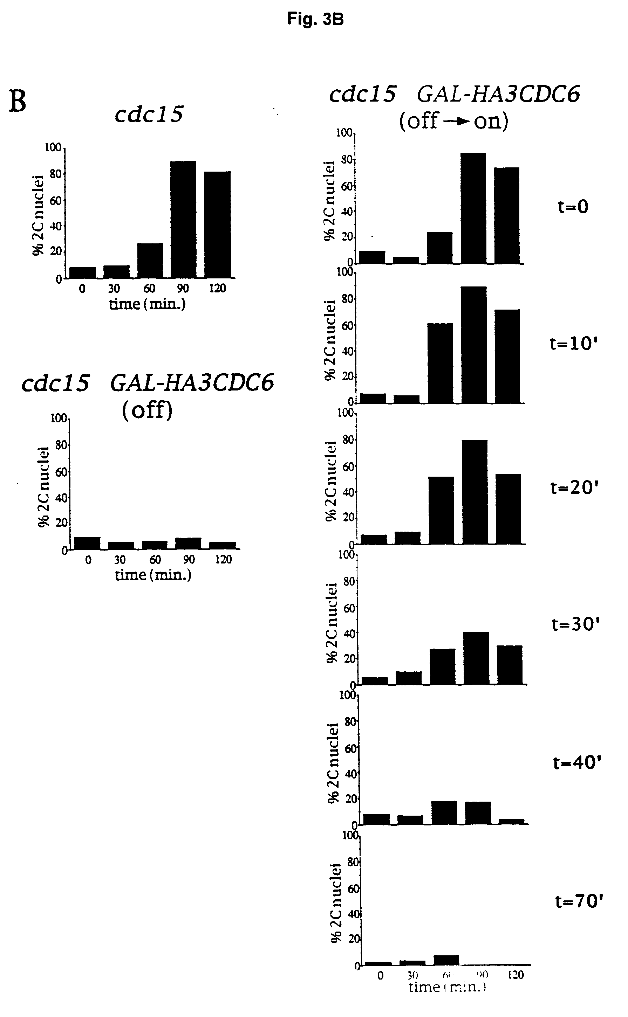

Then the experiment in Fig.2 was repeated varying the time of *GAL-ubiCDC6* induction; that is, cells were arrested at 37°C, Cdc6 synthesis was repressed and re-induced at 0, 10, 20, 30 etc min. after release from the *cdc15* arrest. Fig. 3A shows for each time point the percentage of nuclei that replicated their DNA during the subsequent 120 min. All cells retained the ability to replicate DNA upon addition of galactose until 20 min. after the release, some of the cells lost the ability by 30 min., and few if any cells retained it by 40 min. We repeated this experiment using a strain in which the synthesis of Cdc6 (this time not fused to ubiquitin) was instead controlled by the methionine-repressible *MET3* promoter. Cells again lost the ability to replicate DNA upon induction of Cdc6 synthesis 40 min. after release from the late anaphase *cdc15* arrest (data not shown). These data suggest that there exists a "point of no return", between 30 and 40 min. after *cdc15* release, after which induction of *CDC6* transcription is ineffective in driving DNA replication. It is interesting that this point is around the time that cells would normally have entered S phase, had the *CDC6* gene been fully functional.

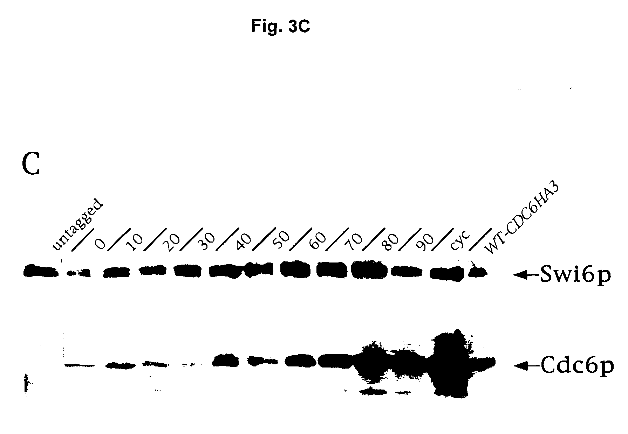

The question was addressed if a cell's failure to induce DNA replication after the "point of no return" might be due to a simple failure to synthesise sufficient Cdc6 protein? It was previously shown that Cdc6 protein made in wild type cells during late G1 is rapidly degraded as cells progress through the cell cycle (Piatti et al., 1995). Thus, Cdc6 protein might fail to accumulate in cells that have passed the "point of no return" due to an increase in the rate of Cdc6 proteolysis at this stage of the cell cycle. Therefore the previous experiment was repeated using a strain whose sole functional *CDC6* gene is an HA tagged version under control of the *GAL1-10* promoter (*GAL-HA3CDC6*). The HA tagged gene is fully functional because these cells proliferate normally in galactose medium. The epitope tag allowed us to follow accumulation of Cdc6 protein by Western blotting 30 min. after induction at various times following *cdc15* release. These cells also lost the ability to replicate DNA in response to galactose induction 40 min. after release (Fig. 3B), even though they retained the ability to accumulate Cdc6 protein throughout the time course (Fig. 3C). In fact, higher levels of HA3Cdc6 accumulated 60 min after *cdc15* release, by which time no cells were capable of replicating, than at 20 min. after release, at which time galactose induced most cells to replicate. Thus, an increased proteolysis of Cdc6 after the "point of no return" is not responsible for the inability of cells that have passed it to replicate DNA in response to *de novo* Cdc6 synthesis.

3) Determination of when Cdc6 executes its function

To address whether Cdc6 is needed for DNA replication subsequent to the formation of pre-RCs, wild type and temperature sensitive *cdc6-1* cells were arrested in α factor at the permissive temperature, incubated at 37°C for 2 hours (under which circumstance pre-RCs disappear (Cocker et al., 1996)), and then released from the pheromone block at 37°C. Wild type cells replicated their DNA synchronously, whereas the *cdc6* mutants replicated extremely poorly, if at all (data not shown). Both cultures budded with similar kinetics. *CDC6* cannot therefore execute its replication function during a pheromone induced G1 arrest, even though cells had previously formed pre-RCs. *CDC6* is therefore necessary for the formation and maintenance of pre-RCs during early G1 when Cdk1 is inactive but also for subsequent DNA replication when Cdk1 is activated in late G1.

4) The "point of no return" depends on activation of S phase promoting CDKs

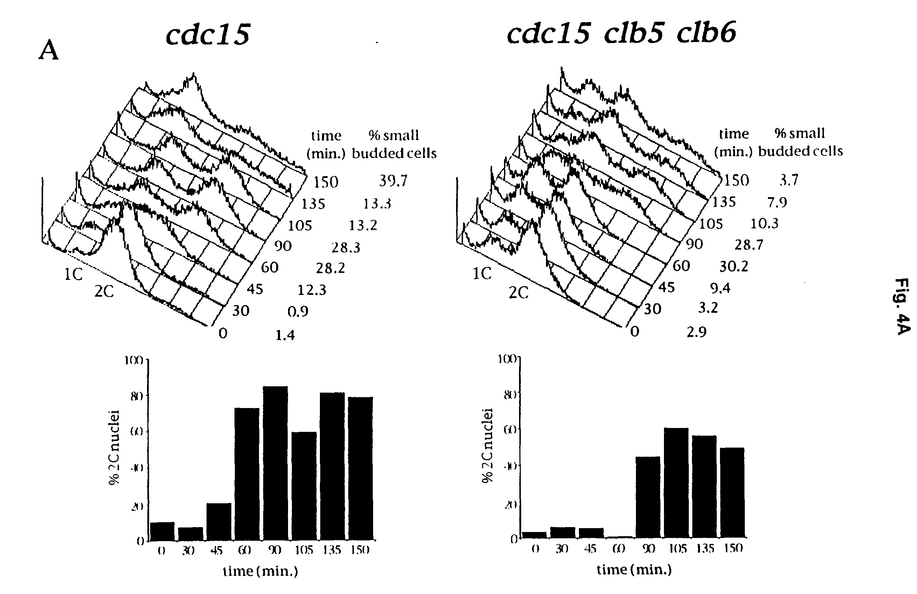

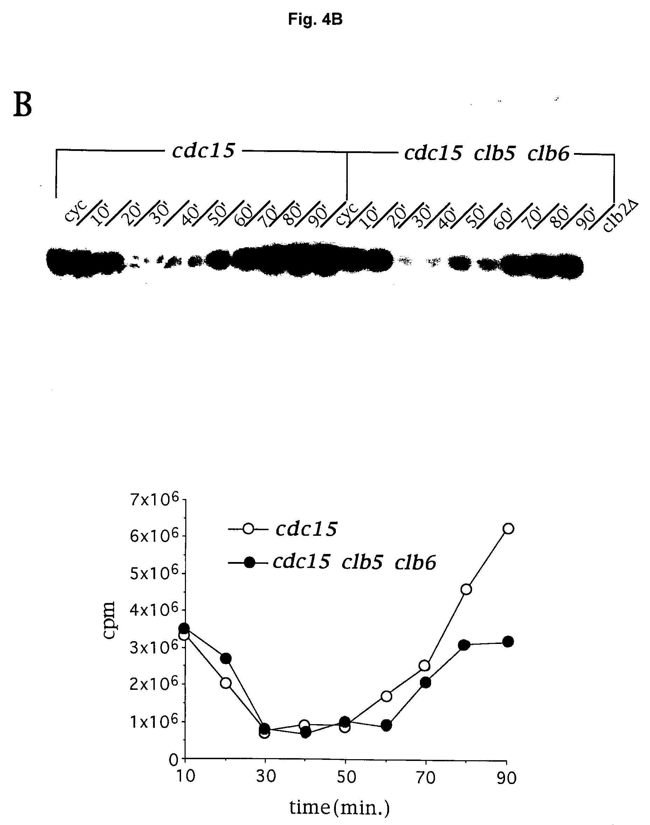

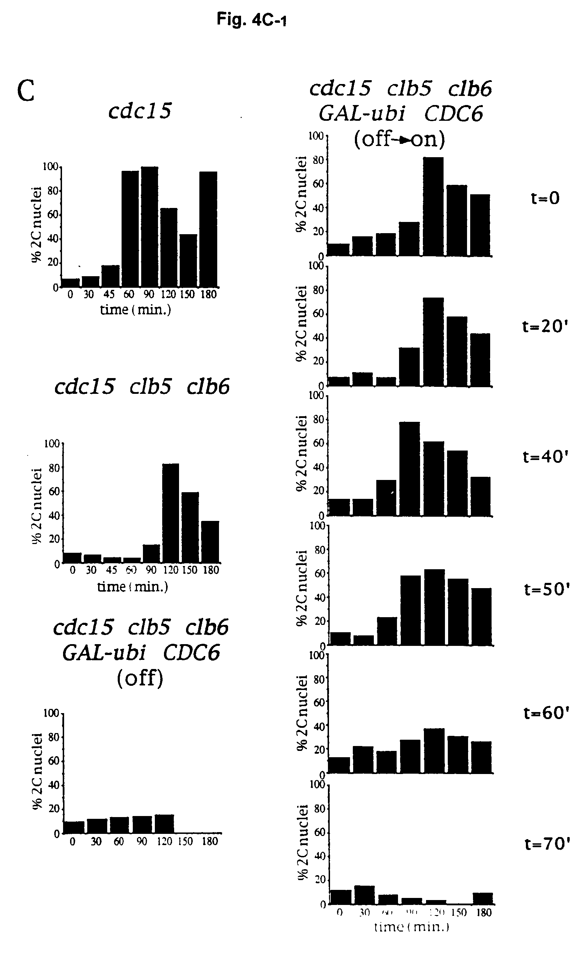

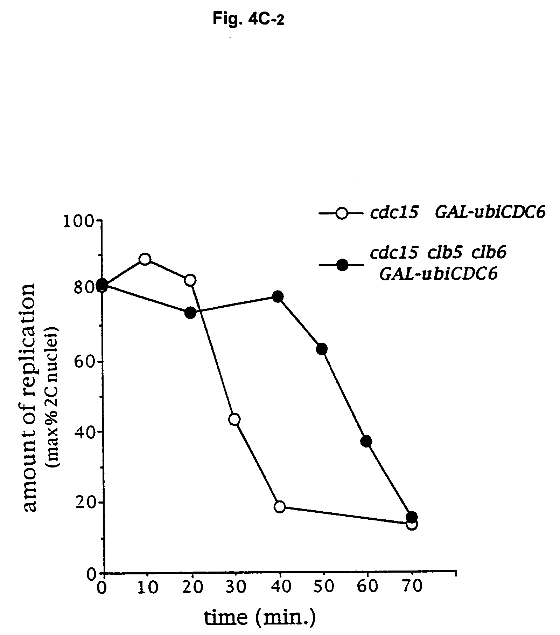

The "point of no return" corresponds roughly with activation of Cln1,2/ and Clb5,6/Cdk1 kinases in late G1. Dahmann et al. (1995) have postulated that Clb/Cdk1 kinases have dual roles: they trigger origins that have previously formed pre-RCs to initiate DNA replication, while they simultaneously prevent *de novo* formation of pre-RCs. If S phase promoting Clb/Cdks were involved in preventing Cdc6 from inducing replication, then inactivation of *CLB5* and *CLB6* should delay the "point of no return", as they are the first B-type cyclin genes to be expressed during the cell cycle (Schwob and Nasmyth, 1993). S phase entry is delayed by about 30 minutes in *clb5 clb6* double mutants; that is, until the later accumulation of Clb1-4/Cdk1 kinases (Schwob et al., 1994). This delay is also seen during release from a *cdc15* arrest. *CLB5 CLB6 cdc15* cells commence replication about 45 min. after their release, whereas *clb5 clb6 cdc15* mutants do not start until 90 min. even though budding and activation of Clb2/Cdk1 kinase occur on schedule (Fig. 4A and B). Deletion of *CLB5* and *CLB6* also extended the length of time, following release from a *cdc15* arrest, during which induction of Cdc6 was capable of promoting DNA replication (compare Figs. 3A and 4C); 50% of *CLB5 CLB6* cells passed the "point of no return" by 30 min., whereas the equivalent point was not reached until 60 min. in *clb5 clb6* double mutants. Very similar results were obtained comparing the "points of no return" of *cdc15 MET-CDC6* and *cdc15 clb5 clb6 MET-CDC6* cells. Thus, loss of Clb5 and Clb6 activity delays passage through the "point of no return" to a degree that is similar to the delay in S phase entry. This finding implies that Cln/Cdk1 kinases, which are also required for S phase entry as activators of Clb kinases (Amon et al., 1994; Dirick et al., 1995), are not sufficient to promote passage through the "point of no return", because the kinetics of their activation is not affected by deletion of *CLB5* and 6. Clbs1-4 assume the S phase promoting function of Clbs 5 and 6 in *clb5 clb6* double mutants. It was presumed that they also assume from Clbs5 and 6 the function of promoting passage through the "point of no return". This presumption is consistent with the finding that inhibition of all six Clb/Cdk1 kinases in nocodazole blocked cells is sufficient to induce pre-RCs (Dahmann et al., 1995).

5) Cdc6p interacts *in vivo* with Clb/Cdk1 kinases

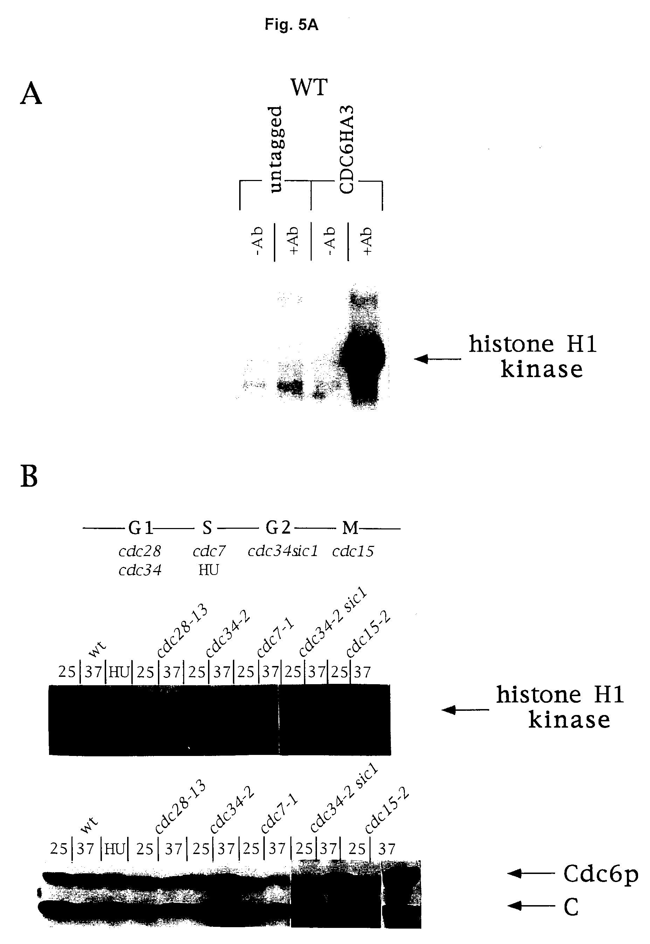

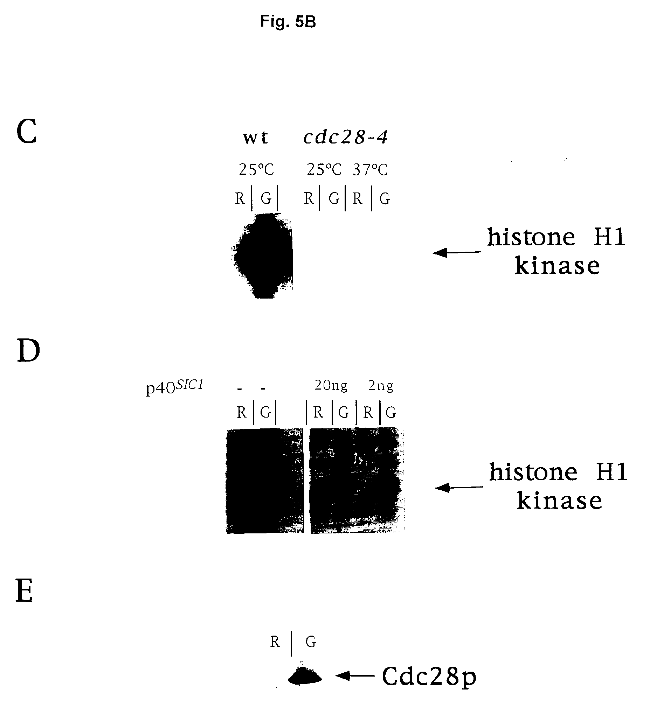

A histone H1 kinase activity co-immunoprecipitates with a version of Cdc6 protein tagged at its C-terminus with the HA3 epitope and expressed from its own promoter (Fig. 5A). The Cdc6-associated kinase in extracts derived from cells of various *cdc* mutants incubated in either permissive or restrictive conditions was assayed and active kinase was found in all extracts except those prepared from *cdc28-13* and *cdc4-1* mutant cells incubated at the non-permissive temperature (Fig. 5B). Western analysis showed that Cdc6p was present at similar levels in these extracts (Fig. 5B). Several data suggest that the activity associated with Cdc6 corresponds to Clb/Cdk1 kinases. First, these kinases are inactive in *cdc4-1* cells due presumably to accumulation of p40$^{SIC1}$ protein (Schwob et al., 1994) and deletion of *SIC1* restored Cdc6-associated kinase activity (Fig. 5B). Second, little or no kinase activity associated with HA tagged Cdc6 expressed from the *GAL* promoter (*GAL-HA3CDC6*) was found in *cdc28-4* cycling cultures (Fig. 5C). Third, the kinase was inhibited by the addition of purified p40$^{SIC1}$ (Fig. 5D). Fourth, Cdc28 protein co-immunoprecipitated with Cdc6p in extracts from *GAL-HA3CDC6* cells (Fig. 5E). These data suggest that Cdc6p made in S, G2 or M phases associates with Clb/Cdk1 kinases. This association might be instrumental in preventing Cdc6 from forming pre-RCs at origins during these stages of the cell cycle. The "point of no return" might therefore correspond to the point in the cell cycle at which Cdc6 protein, if present or produced, associates with Clb/Cdk1 kinases. It is not yet known whether Cdc6p is a target for these kinases *in vivo* ; it can, however, be phosphorylated by Clb/ but not by Cln/Cdc28 kinases *in vitro* (data not shown).

From the above results, the following conclusions can be drawn:

1) Cdc6's ability to promote formation of pre-RCs and DNA replication is restricted to the G1 phase of the cell cycle

*De novo* Cdc6 protein synthesis is required for a change in the chromatin structure of origins of DNA replication, which normally occurs as cells exit from mitosis and enter G1 (Cocker et al., 1996). The pattern of DNaseI sensitivity surrounding origins suggests that the Origin Recognition Complex (ORC) binds to origins throughout the cell cycle but that another factor, possibly Cdc6 itself, joins it in the interval between exit from mitosis and re-initiation of DNA replication in the subsequent cycle (Diffley et al., 1994). Cdc6 protein has been shown to interact with ORC in vitro (Liang et al., 1995). Cdc6 is required for the initiation of DNA replication but it is not required for other aspects of cell cycle progression such as the formation of mitotic spindles or even the movement on these spindles of unreplicated chromosomes to opposite poles of the cell (Piatti et al.,1995). These findings suggest that a change in the state of replication origins mediated by Cdc6 synthesis at the end of mitosis or later during G1 is essential for the subsequent initiation of DNA replication triggered by activation of Clb/Cdk1 kinases in late G1. The initiation of DNA replication in yeast can therefore be viewed as a two step process: formation at origins of pre-replication complexes (pre-RCs), driven by Cdc6 synthesis, followed by activation of Clb/Cdk1 kinases due to destruction of the Clb-specific Cdk inhibitor p40$^{SIC1}$ (Schwob et al., 1994). The experiments of the invention address whether these two processes must occur in the correct order. Clb/Cdk1 kinases are activated on schedule even when Cdc6 protein is not made during G1. What happens then when Cdc6 synthesis is restored to cells that have already activated Clb/Cdk1 kinases? Is this effective in promoting DNA replication? The effectiveness of Cdc6 synthesis at different cell cycle stages in promoting DNA replication was investigated and a "point of no return" was discovered after which *de novo* Cdc6 synthesis fails to drive DNA replication. This "point of no return" is delayed by inactivation of Clb5 and Clb6, which are normally the first Clbs to appear during the cell cycle. Passage through the "point of no return" did not, however, depend on activity of the Cdc7 protein kinase (data not shown), which is also necessary for initiation of DNA replication and for the switch of origins from a pre-replicative to a post-replicative state. The data suggest that the "point of no return" corresponds to a point in the cell cycle after which Cdc6 protein is no longer able to drive the formation of pre-RCs at origins of DNA replication and that the crucial event responsible for this switch in cell cycle state is the activation of Clb/Cdk1 kinases. The "point of no return" not only coincides with Clb/Cdk1 activation but is also delayed when their activation is delayed.

Dahmann et al. (1995) showed that inactivation of Clb/Cdk1 kinases in cells arrested in nocodazole by overproduction of the p40$^{SIC1}$ Cdk inhibitor is sufficient to induce the formation of pre-RCs at origins. As a result, they proposed that Clb/Cdk1 kinases have two opposing roles in the initiation of S phase: to inhibit the *de novo* formation of pre-RCs and to trigger initiation only from those origins that have previously formed pre-RCs. The re-replication of S. pombe cells either defective for Cdc2 or the B-type cyclin Cdc13, or overproducing the Cdc13/Cdc2 kinase inhibitor Rum1, is consistent with this hypothesis (Broek et al., 1991; Hayles et al., 1994; Moreno and Nurse, 1994). According to this model, activation of Clb/Cdk1 kinases prior to the formation of pre-RCs (i.e. prior to the *de novo* synthesis of Cdc6 protein) would finesse the system and prevent initiation of DNA replication. The results described above show that the timing of Cdc6 synthesis relative to Clb/Cdk1 activation is indeed crucial. The "point of no return" may correspond to the point in the cell cycle when Clb/Cdk1 kinases become sufficiently active to inhibit the formation of pre-RCs at origins.

The experiments of the invention concentrated on the consequences of delaying Cdc6 synthesis relative to Clb/Cdk1 activation. However, the hypothesis that Clb/Cdk1 kinases inhibit the formation of pre-RCs predicts disaster not only when Cdc6 synthesis is delayed but also when Clb/Cdk1 kinases are activated prematurely. In other experiments the effects on replication of deleting the *SIC1* gene, which encodes an inhibitor of Clb/Cdk1 kinases that delays their activation during G1, have been analysed. *sic1* mutants initiate DNA replication somewhat earlier than wild type cells but proceed through S phase more slowly. Furthermore, chromosomes and mini-chromosomes are highly unstable in *sic1* mutants and this defect is suppressed either by deletion of *CLB5* and *CLB6* or by the addition of extra origins to the chromosomes. Thus, premature activation of Clb/Cdk1 kinases has a similar effect to delaying Cdc6 synthesis: it also reduces the efficiency with which origins fire.

2) Factors that prevent the formation of pre-RCs during G2

The lack of DNA replication in cells that express *GAL-CDC6* only after they have passed the "point of no return" is not due to their failure to accumulate sufficient Cdc6 protein; induction of *GAL-CDC6* causes more Cdc6p to accumulate in cells that have long passed this point than in those that have not (Fig. 3C). Cdc6p is an unstable protein and it normally disappears from cells during S and G2 phases, but this is primarily due to control of *CDC6* transcription. Under conditions in which Cdc6 was synthesised continuously and protein levels remained constant throughout the cycle (due to expression from the *GAL* promoter), it could be noticed that more Cdc6 protein accumulated within nuclei of post anaphase and early G1 cells than during S, G2, and M phases (Piatti et al., 1996). Rapid proteolysis of Cdc6 seems partly responsible for this pattern because Cdc6 protein accumulated to high levels within nuclei at most stages of the cell cycle in cycling cultures of *cdc4* mutants, which are defective in Cdc6 degradation (Piatti et al., 1996).

The cell cycle control of Cdc6's cellular distribution resembles that of Cdc46/Mcm proteins, which are also needed for the firing of origins and which also accumulate in the cytoplasm during G2. It is doubtful, however, whether Cdc6's failure to function in cells past the "point of no return" is due to its insufficient accumulation within nuclei. Cdc6 protein is never actually excluded from nuclei in *CDC4*$^+$ cells and it accumulates to very high levels within G2 nuclei of *cdc4* mutant cells growing at 25°C without bypassing the block that prevents re-replication until cells have undergone anaphase (Piatti et al., 1996). The results of the experiments raise the question as to why Cdc6 is only synthesised during G1 and is rapidly degraded in wild type cells. This seems to be a conserved feature of the fungal cell cycle, because Cdc18p levels in S.pombe are regulated in a similar manner (Nishitani and Nurse, 1995; Muzi-Falconi et al., 1996). It has been shown that the block to re-replication does not depend on Cdc6's absence from the nucleus during G2. It might nevertheless contribute to the fidelity with which such cells prevent re-replication.

Cdc6's inability to promote DNA replication in cells that have past the "point of no return" could be due either to its association with inhibitory factors or due to its inhibition by modification. The finding that Cdc6 associates with Clb/Cdk1 kinases could be important with this regard. Phosphorylation of Cdc6 by these kinases might prevent it from forming pre-RCs. Alternatively, Clb/Cdk1 kinases associated with Cdc6 might inhibit, through phosphorylation, other Cdc6-interacting proteins needed for pre-RC formation (for example, members of ORC).

The failure of cells past the "point of no return" to form pre-RCs and to promote replication in response to *de novo* Cdc6 synthesis could also stem from the inactivity in these cells of other proteins needed to form pre-RCs or to initiate DNA replication from them. For example, proteins of the Cdc46/Mcm class, which are essential for origin firing, are found in the cytoplasm during G2. Thus, a drop in the concentration of Mcm proteins within nuclei or a reduction in their ability to bind chromatin might be responsible for the "point of no return".

3) Factors that drive the eukaryotic replication cycle

A key requirement for the mechanism that drives chromosome duplication is that it should permit replication origins to fire once and once only once between succeeding rounds of sister chromatid segregation. Bacteria must also restrict replication to once every mass doubling, but the mechanism by which they do this is different due to their possession of only a single origin of DNA replication. Imperfections in the bacterial firing mechanism are not disastrous because unscheduled initiation from a single origin leads to replication of the entire genome; that is, it does not lead to over or under representation of genes. The downside of this simple device is that replication can take longer than mass doubling and this problem can only be solved by re-initiating replication before sister chromatids from the previous round have been segregated. The mechanism used by eukaryotes whose genomes are carried on multiple chromosomes, each of which are replicated from multiple origins, must therefore be much more efficient than that used by bacteria. There has been much speculation as to how eukaryotic cells might achieve an efficient "once only" firing device, but few hard facts.

The current knowledge about replication in S. cerevisiae is sufficient to assemble, for the first time, the crude outlines of the device actually used (see Fig. 6 and Dahman et al., 1995). Initiation is from defined sites and depends on two types of factors: those like ORC and, presumably, Cdc6 and Cdc46/Mcm proteins that bind to origins or the sequences that surround them and S phase promoting factors like Clb/Cdk1 and Cdc7 kinases, whose activation in late G1 actually triggers the initiation of DNA replication. Origins and their surrounding chromatin exist in two states: a post-replicative one, in which they are bound by ORC alone, and a pre-replicative one, in which they are bound by ORC and possibly also Cdc6 and Mcm proteins (Diffley et al., 1994; Cocker et al., 1996). We refer to the latter as the pre-replication or pre-initiation complex. Only origins in a pre-replicative state can be triggered to initiate DNA replication by Clb/Cdk1 kinases. Key aspects of this "once only" replication device are first the mechanisms that govern the transitions between the two states of origins and second the mechanisms that generate sharp fluctuations in the activity of Clb/Cdk1 kinases. It is proposed that pre-RCs are destroyed either by initiation itself or by replication through them and that the formation of new pre-RCs is inhibited by the very same set of Clb/Cdk1 kinases that trigger initiation. According to this scheme, the replication cycle is driven by a Clb/Cdk1 cycle. It starts with a period of low Clb/Cdk1 activity (G1), which permits formation of pre-RCs. Activation of Clb/Cdk1 kinases triggers replication from pre-RCs that had formed during the previous period of low Clb/Cdk1 activity and simultaneously blocks formation of any new pre-RCs (S phase). The subsequent period of high Clb/Cdk1 kinase activity (G2/M phases) maintains the block to the formation of pre-RCs. The cycle is completed by the final inactivation of Clb/Cdk1 kinases and re-entry into the low kinase state, which is mediated, at least in part, by the same complex (the APC) that promotes the segregation of sister chromatids (Irniger et al., 1995). The antagonistic effects of Clb/Cdk1 kinases on initiation ensure that there is no stage during this cycle in which cells can both form pre-RCs and trigger initiation of DNA replication from them. Involvement of the APC in both cyclin destruction and in promoting sister chromatid separation may be key to linking re-replication with chromosome disjunction.

The momentum of the device, i.e. successive rounds of DNA replication, can only be maintained by fluctuations in the activity of Clb/Cdk1 kinases. It is a reciprocating device in which motion (i.e. rounds of replication) is driven by the alternate inactivation and re-activation of Clb/Cdk1 kinases. In this sense, it is deeply analogous to the reciprocating steam engine, whose steam corresponds to kinase activity and whose piston corresponds to origins. Steam, i.e. kinase, can only do work by driving the piston upwards (i.e. drive replication) if it (i.e. origins) has previously returned to the down state (i.e. contains pre-RCs). Passage from the up state (an origin that has initiated replication) depends on evacuation of the steam (i.e. inactivation of kinases) from the chamber containing the piston (i.e. the cell). The essence of the reciprocating steam engine is that entry of steam into the chamber only performs work if the piston has been returned to the down state by previous evacuation of steam from the chamber. Likewise, Clb/Cdk1 kinases only drive replication when their prior inactivation has permitted the formation of pre-RCs. Just as the piston can only move once during a cycle of expansion and contraction, so too can origins only fire only once during a cycle of kinase activation and inactivation. If then, there exists a cell cycle engine (Murray and Hunt, 1993), this surely could be a key part of it.

Timing is crucial to the operation of the "reciprocating" steam engine. The implication of the finding that Cdc6 must be synthesised before Clb/Cdk1 kinases are re-activated suggests that timing is also crucial to the "reciprocating" replication cycle in S. cerevisiae.

It was an object of the invention to provide compounds for use in cancer therapy.

The experiments of the present invention have shown that the initation of DNA replication in eukaryotic cells, exemplified with yeast cells, is a two step process: first the formation at future replication origins of pre-replication complexes and second the activation of S phase promoting cyclin dependent kinases (Diffley et al., 1994; Schwob et al., 1994; Cocker et al., 1996). Synthesis of the unstable protein Cdc6 is essential for the formation of pre-RCs at yeast origins and for subsequent initiation of DNA replication.

The present invention is based on results of an investigation of when during the cell cycle synthesis of Cdc6 is effective in forming pre-replication complexes and thereby in driving a new round of DNA replication. The experiments of the present invention have shown that Cdc6 must be synthesised during the period of the cell cycle in which S phase and M phase promoting cyclin dependent kinases are inactive (i.e. G1 phase). It has been shown that synthesis of Cdc6 after these kinases have become active is ineffective in driving a new round of DNA replication. Cells deprived of Cdc6 proceed with the activation of S and M phase promoting kinases and enter mitosis and even attempt to undergo anaphase without having undergone DNA replication, which is a lethal event (Kelly et al., 1993; Piatti et al., 1995). The key conclusion that can be drawn from these results is that a temporary interference with Cdc6 function, during a cell cycle period in which S or M phase promoting cyclin dependent kinases become active, is sufficient to kill proliferating cells but has no effect on quiescent cells.

The present invention provides a method of interfering with the proliferation of tumour cells. This method is based on a novel concept which allows for identifying drugs which are toxic to the tumour cells, i.e. rapidly growing cells, but which are harmless to the non-dividing or slowly dividing non-tumour cells of the patient being treated with that drug.

The present invention provides a method to kill tumour cells which is based on the utilization of this novel observation.

While it has been known since the first CDC6 mutations were isolated (Hartwell, 1976), that CDC6 is essential for proper DNA replication and a loss of function of the CDC6 gene results in cell cycle arrest, the experiments of the present invention show for the first time that an interference with Cdc6 function only for a short period of time is also a lethal event for cells that are in the process of activating cyclin dependent kinases (Cdks) that drive DNA replication and mitosis. The experiments of the invention also suggest a mechanism for this phenomenon. Thus, the application of a drug that interferes with the ability of Cdc6 to form or maintain pre-RCs (or a drug that destroys pre-RCs) is lethal to cells which proceed to activate S and M phase promoting Cdks in the presence of the drug, even when the drug has been removed). The repeated temporary application, e.g. for 12 to 24 hours, of drugs that interfere with the ability of CDC6 to form or maintain pre-replication complexes will be lethal to rapidly growing tumour cells which activate S and M phase promoting cyclin dependent kinases in the presence of the drug, but will have little or no effect on quiescent or even slowing dividing host cells which spend much longer in a G1 state where these kinases are inactive. The results of the experiments of the present invention provide a proof of this principle in the budding yeast *Saccharomyces cerevisiae*. It has been demonstrated that depriving cells of Cdc6 function merely for 40 minutes, between their exiting mitosis and re-activating S phase promoting Cdks in the next cycle, is a lethal event.

Since most of the components directly involved in the initiation of DNA replication, for example, S phase promoting cyclin dependent kinases, the origin recognition complex (ORC), Cdc6, and Mcm proteins, which are DNA binding proteins, have proven to be highly conserved between fungi and animal cells, this proof of principle in budding yeast according to the results of the experiments of the present invention is highly relevant to human cells. The present invention is based on the validity of the assumption that the principles about Cdc6 function and the assembly of pre-replication complexes, or their inhibition, respectively, that have been discovered in yeast are also valid for human cells. The identification of homologous genes in as distantly related organisms as yeast and Xenopus has so far always led to the identification of a homologous mammalian gene. Therefore, a recent report on the Xenopus homologue of Cdc6 (Dunphy, W., Meeting "The Cell Cycle", Cold Spring Harbour, May 1996) is a proof that DNA replication in higher eukaryotic cells, and also in human cells, also depends on the presence and activity of Cdc6 during G1 phase when S and M phase promoting Cdks are inactive. Temporary inhibition of the cell's ability to assemble pre-replication complexes is therefore as lethal to rapidly dividing human cells as it is to proliferating yeast cells.

Replication origins have not yet been fully characterized in mammalian cells and may prove to differ, in certain aspects, to yeast origins. Nevertheless, based on the reported conservation of replication mechanisms in eukaryotic cells, it has to be assumed that they will operate according to similar principles that involve homologous protein functions.

The invention provides the use of compounds which interfere with the function of cdc6p to form or maintain pre-replication complexes in an animal cell without impairing the cell's ability to activate cyclin dependent kinases that promote S phase and/or M phase, for the treatment of cancer.

In the following, compounds which interfere with the function of cdc6p to form or maintain pre-replication complexes, are also designated "cdc6p inhibitors".

In exploitation of the above-described concepts, the present invention further provides screening methods for identifying compounds for treating cancer that can interfere with Cdc6-mediated pre-replication complex formation and maintenance in animal cells.

In one embodiment of the invention, this screening method is based on the detection of the formation of pre-replication complexes. An example is an assay in which the binding of Cdc6 protein to chromatin is detected by antibodies directed against Cdc6.

The yeast cdc6 protein having been cloned (Zhou et al., 1989), based on the published yeast sequence the homologous human protein can be obtained by methods known in the art, e.g. by screening human cDNA libraries with DNA probes derived from the yeast sequence or by PCR techniques. An alternative method to obtain the human CDC6 cDNA is the functional complementation of the cdc6(ts) allele in yeast by expression of the homologous human CDNA according to methods konwn in art, designated "expression cloning". The human CDC6 cDNA may then be expressed according to known methods in suitable hosts (Maniatis et al., 1982). The thus obtained human cdc6 protein or peptides derived from its amino acid sequence may then be used to obtain antibodies according to known methods, preferably monoclonal antibodies, which are produced by conventional hybridoma technology (see e.g. Harlow and Lane, 1988).

Since the binding of Cdc6 may be a pre-requisite for the binding of Mcm proteins to chromatin, Cdc6 activity may also be measured indirectly by measuring the binding of Mcm proteins to chromatin.

An assay for identifying cdc6p inhibitors by detecting the formation of pre-replication complexes may be performed using live human cells. To this end, rapidly dividing cells, e.g. cells from a tumour cell line, are grown and incubated with the test substance for a period of time sufficient for cdc6p's binding to chromatin. Then the cells are lysed, fixed on cover slips and the binding of cdc6p to chromatin is determined as described above. The use of synchronized cells, which are at a stage of the cell cycle prior to cdc6p synthesis, is advantageous.

Since the drugs of interest must be specific for an activity that is present only during a narrow window of the cell cycle, an *in vitro* system which provides this very window is also useful for identifying cdc6p inhibitors. Such a screening assay may be performed as a microtitre plate assay, in which a Xenopus laevis egg extract (Blow,J.J. and Laskey, R.A., 1988), which is capable of DNA replication and therefore also contains cdc6p, and chromatin, e.g. Xenopus laevis sperm chromatin, is incubated, in the presence of the test substance, for a period of time sufficient for cdc6p to form pre-replication complexes. After the incubation period assembly of Cdc6 protein onto chromatin can be measured by simple immunological methods, e.g. *in situ* immunofluorescence, employing anti-cdc6 antibodies carrying an immunofluorescent label. References for X.L. extract capable of replication.

The use of Xenopus components in an assay system is extremely useful in proving the potentional drug's efficacy in animal cells. Compounds identified as inhibitors of cdc6p's binding to chromatin in a screening assay using the *in vitro* Xenopus laevis system can be rapidly tested on Xenopus tissue culture cells to confirm their effect on rapidly cycling somatic cells.

It would, however, be preferable to test compounds using an *in vitro* assay for the formation of pre-RCs in extracts from human cells. To do this, sperm chromatin or nuclei isolated from G1 cells would be added to extracts prepared from human cells that express high levels of cdc6p and a cyclin dependent kinase inhibitor such as P21 or P27.

If the human protein were found to rescue the lethality of a yeast cdc6 mutation, then yeast cells can be employed to assay the function of the human cdc6 protein in yeast cells.

An example for a yeast based screening assay for identifying cdc6p inhibitors, which can be used according to the invention, is based on the sort of experiments conducted by Liang et al., 1995, who have shown that cdc6 is a multisuppressor of orc mutations in yeast, i.e. the cells carrying an orc mutation require overexpression of cdc6p for their growth. Thus, *Saccharomyces cerevisiae* cells that carry an orc mutation, e.g. the orc-5 or orc-2 mutants described by Liang et al. 1995, and an inducible, e.g. galactose inducible, plasmid containing the, CDC6 sequence (preferably the human sequence) such that they overexpress cdc6p, may be used in a screening assay. The yeast cells are grown and incubated with the test substances. Substances that cause the cells to die are candidates for inhibitors of cdc6 function.

Another means of assaying the function of cdc6 homologues (and thereby screening for drugs that interfere with that function) is based on the ability of CDC6/cdc18 homologues to induce multiple rounds of DNA replication, in the absence of mitosis, in the fission yeast *Schizosaccharomyces pombe* (Muzi-Falconi, et al., 1996, Nishitani und Nurse, 1995). Inhibition of cdc6 function by a test substance will abolish the increase in DNA content, which can be measured by conventional means, e.g. by staining with DAPI (4,6-diamido-2-phenylindole-dihydrochloride).

To narrow the results of the above screening methods to drugs which, while blocking cdc6 function, do not impair the activity of cyclin dependent kinases, it is necessary to do controls. Suitable controls show that these kinases are still active and can be activated in the presence of the drug that interferes with cdc6 function. Such control assays may be based on the detection of kinase activity (CDK2), e.g. by H1 histone phosphorylation, and/or the accumulation of these kinases' relevant cyclins (Cyclins E and A) with anti-cyclin antibodies carrying an immunofluorescent label. The controls may be carried out in parallel with the primary screen. Preferably such controls are carried out in a second screening step. Chemical compounds that inhibit the binding of cdc6 to chromatin while they retain kinase activity and/or do not prevent the cells from accumulating the relevant cyclins (ClnE, ClnE) fulfil the requirement of blocking cdc6 activity while leaving kinase function and activation unimpaired.

It is expected that the screening methods of the invention that are based on the detection of pre-RCs will identify not only drugs that interfere with cdc6p function, but also drugs that cause S phase promoting Cdks to become active prematurely. Such drugs are expected to interfere with Cdk inhibitory proteins and might be equally toxic to rapidly dividing tumor cells, because the premature activation of Cdks would also interfere with pre-RCs..

In parallel to drug screening, it is useful to conduct the sort of experiments of the present invention, that were performed with yeast, in animal cells. This may be accomplished by replacing the endogenous CdC6 gene of mouse embryonic stem cells by a version of the gene whose transcription can be induced, i.e. be turned on and off at will, for example using the tetracycline system. A prerequisite for the feasibility of this type of experiment is that there proves to be a single copy of the mouse Cdc6 gene. This can be verified by standard methods such as Southern hybridization.

It has to be borne in mind that the drugs identified according to the invention are inhibitors against cdc6 function and may thus be toxic to other cells that are, at the time the drug is exerting its effect, performing a rapid cell cycle. To prevent undesired side effects on such cells, i.e. stem cells, particularly those from bone marrow, like erythrocyte and thrombocyte progenitors, an additional drug may have to be applied that interferes with signalling pathways specific for such cells such that the activation of S phase promoting kinases is prevented without affecting tumour cells, and the non-tumour cells are arrested in G1 phase of the cell cycle, while the cdc6p inhibitor does its effect. Treatment of a patient with both drugs would block stem cells in a low kinase state and would enable them to assemble pre-RCs once the anti-Cdc6 drug is no longer active, but it would not compromise the lethality of the anti-cdc6 drug to inhibit pre-RCs in tumour cells. A possible example would be to use immunosuppressant drugs like cyclosporin which are, due to their mode of action, likely to be more effective in blocking bone marrow cells in a low kinase state than they are in blocking tumour cells in such a state. This concept of protecting normal cells has been proven in the yeast experiments of the invention which showed that blocking yeast cells in a low cyclin dependent kinase state with $\alpha$-factor prevents cells from passing the "point of no return" and thereby alleviates the lethality associated with blocking synthesis of Cdc6.

Brief description of the drawings

Fig. 1:      Pre-RCs cannot be formed in G2/M

Fig. 2:      Cells deprived of Cdc6 in late mitosis are unable to replicate in the next cell cycle but undergo reductional" anaphase

Fig. 3:      Cells pass a "point of no return" at the time of S phase entry after which Cdc6 synthesis is unable to promote DNA replication

Fig. 4:      Deletion of *CLB5* and *CLB6* causes a delay in S phase entry and a similar delay in the "point of no return

Fig. 5:      B-type cyclin dependent Cdc28 kinase associates with Cdc6p *in vivo* during S, G2 and M phases.

Fig. 6:      The pre-RC/Cdk1 cycle in yeast

If not otherwise indicated, the following materials and methods were used in the Examples:

i) Strains, media and reagents

All yeast strains were derivatives of or were backcrossed at least three times to W303 (*HMLa, HMRa, ho, ade2-1, trp1-1, leu2-3,112, his3-11,15, ura3, ssd1*). Cells were grown in YEP medium (1% yeast extract, 2% bactopeptone, 50 mg/l adenine) supplemented as indicated with 2% glucose (YEPD), 2% raffinose (YEPR) or 2% raffinose +2% galactose (YEPRG), except for the experiment described in Example 2 B and C, where 0.1% galactose was added to YEPR medium. The synthetic medium lacking methionine (-Met medium) is yeast nitrogen base (0.8%) supplemented with amino acids, adenine, uracil and 2% glucose.

ii) Plasmid constructions and genetic manipulations

Standard techniques (Mortimer and Hawthorne, 1969) were used for genetic crosses and DNA manipulations (Maniatis et al., 1982).
To generate the *GAL-ubiCDC6* construct (C2835), the ATG of *CDC6* has been replaced by a BamHI site introduced by PCR. Afterwards, a 2 kb BamHI/(NdeI)HindIII fragment of *CDC6* has been used to replace the *MCM1* fragment of a *GAL-ubiR-MCM1* fusion in YIplac211 (a kind gift from Gustav Ammerer). The resulting plasmid has been cut with ApaI for integration in single copy at the *URA3* locus of W303. This strain was then crossed to K4055 (*cdc6::hisG, trp1::TRP1 MET-CDC6*, Piatti et al., 1995) to generate, after sporulation, strain K4675 (*cdc6::hisG, ura3::URA3 GAL-ubiCDC6*). K4675 was crossed to a *cdc15-2* strain (K1994) or to a *cdc7-1* strain (K2033) to generate, after sporulation, strains K5032 (*MATa, cdc15-2, cdc6::hisG, ura3::URA3 GAL-ubiCDC6*), K5033 (*MATa, cdc15-2, cdc6::hisG, ura3::URA3 GAL-ubiCDC6*) and K5029 (*MATa, cdc7-1, cdc6::hisG, ura3::URA3 GAL-ubiCDC6*).
The C-terminal HA3 tagged version of *CDC6* used here is similar to the one described before (Piatti et al., 1995), with the exception that it does not contain His tags (plasmid C2838). C2838 was integrated in single copy at the *CDC6* locus of W303 to generate a full length tagged version of *CDC6* flanked by a truncated untagged version of the gene (strain K4528). K4528 was crossed to various *cdc* mutants to generate, after sporulation of the corresponding diploids, the strains used in Example 5).
The *GAL-HA3CDC6* (C2837) construct was made by inserting the triple HA NotI cassette (Tyers et al., 1992) after the ATG of *CDC6*, where a NotI site was introduced by PCR. The tagged gene (ending at the NdeI site of *CDC6*) was then cloned in Yiplac211 downstream of a *GAL1-10* promoter containing 70 bp of *CLB5* leader sequence. Subsequently, the plasmid was cut with either BclI for integration at the *CDC6* locus (in one copy, strain K5095, or in five copies, strain K4527), or StuI for integration at *URA3* (strain K5761). K5761 was crossed to K4143 (*cdc15-2, cdc6::hisG, trp1::TRP1 MET-CDC6*) to generate, after sporulation, strain K5763 (*cdc15-2, cdc6::hisG, ura3::URA3 GAL-HA3CDC6*).

iii) Cell Synchronization Techniques

To perform *cdc15-2* block/release experiments, cells were grown to exponential phase, filtered and inoculated into prewarmed medium. Cell cycle arrest was obtained by 3 hours incubation at 37°C and then cells were filtered again and released at 25°C. In the cases where *CDC6* synthesis is controlled by the *GAL1-10* promoter (*GAL-ubiCDC6* or *GAL-HA3CDC6*), cells were harvested by filtration and incubated in prewarmed YEPR medium at 37°C for 30 minutes (*GAL* promoter off) before release in YEPR at 25°C. For induction 2% galactose (or 0.1% in the experiment described in Example 3B and C) was added to aliquots of cells at the different time points.

In the cases where *CDC6* expression was driven by the *MET3* promoter (*MET-CDC6*), cells were treated as above with the exception that the permissive and non-permissive conditions for *CDC6* synthesis were obtained by the use of respectively -Met medium and YEPD+2% methionine.

Due to a cell separation defect displayed after release from the *cdc15* block, some of the cells start replicating before cell division, producing a 4C peak in the FACS profiles, while some others divide before entering S phase. Cells with a 4C DNA content must be binucleate, while the ones with 1C DNA content must be uninucleate. Cells with 2C DNA content can be either bi- or uninucleate. Therefore, to quantify more accurately the fraction of cells able to undergo DNA replication, at each time point after release (up to 2 hours) we have scored by FACS the fraction of cells with 1C, 2C or 4C DNA contents and on the same cell samples the fraction of uni- versus binucleated cells (as determined by immunofluorescence on the propidium iodide stained cells). This allowed us to determine the percentage of 2C nuclei (i.e. nuclei which have replicated) by using the following equation:

$\%2C = 100.(2.C_4 + U - C_1)/(U + 2.B)$, where $C_1$, $C_2$, and $C_4$ are the fraction of cells at each point with 1C, 2C, or 4C DNA contents, and U and B the fractions of uni- and binucleated cells respectively. We can assume that the result of this equation reflects reliably the fraction of nuclei which have replicated during the S phase immediately after *cdc15* release, but its application during later stages of the cell cycle has some limitations: in fact, after completing S phase a fraction of cells undergoes mitosis before having completed the previous cell separation, producing cells with more than 2 nuclei. Since these cells have been scored as binucleates for simplicity, when these cells start appearing the percentage of 2C nuclei becomes overestimated, because some of the cells with a 4C DNA content have four 1C nuclei rather than two 2C nuclei.

Another limitation arises from the feature of Cdc6 deprived cells to undergo anaphase in the absence of DNA replication: in this case, the assumption that 1C cells must be uninucleate does no longer hold true. In cells lacking Cdc6, calculations are even more complicated by the appearance of cells with <1C DNA contents. Therefore, if the formula described above is applied to these cells, the result can be a negative number. In conclusion, we can say that the above equation can be reliably applied only to estimate the fraction of nuclei which have undergone DNA replication during the first S phase after *cdc15* release.

For nocodazole arrest/release experiments described in Fig. 6 A and B, cells exponentially growing in YEPRG were incubated with 5mg/ml nocodazole (and a final concentration of 1% DMSO) at 25°C for 2.5 hours. Cells were then filtered, washed with 3 volumes of YEPR +1% DMSO and incubated in either YEPR +2 mg/ml α factor at 25°C (Fig. 6A) or YEPR at 37°C (Fig. 6 B) for 3 hours.

Afterwards, cultures were split in two and one half was incubated in the presence of 2% galactose for 15 minutes. Subsequently, cells were released by filtration from the α factor or the *cdc7* blocks into either YEPRG or YEPR medium at 25°C for 2 hours.

*iv) In vivo* footprinting

K5033 cells (*MATa, cdc15-2, cdc6::hisG, ura3::URA3 GAL-ubiCDC6*) exponentially growing in YEPRG were arrested by 3 hours incubation at 37°C and then released into YEPD+α factor (10mg/ml) for 90 minutes. An aliquot of the culture was then transferred into YEPRG+α factor, while another was released from α factor into YEPD+nocodazole (20 mg/ml). After 90 minutes cell samples were withdrawn for *in vivo* footprinting and part of the culture containing nocodazole was transferred to YEPRG+nocodazole for 90 minutes. From each condition footprinting experiments on the 2m origin have been performed according to Diffley et al., 1994, on 50 ml of culture ($2x10^7$ cells/ml).

v) Northern and Western Blot Analysis

The methods described by Cross and Tinkelenberg (1991) and Price et al. (1991) were used for RNA isolation and Northern blot analysis respectively.

For Western blot analysis, protein extracts were prepared either as described in Surana et al. (1993) (Fig. 2C and 4B), or by TCA precipitation (Foiani et al., 1994) (Example 5B). 50-150 mg of total extracts were transferred to Immobilon P membranes (Millipore).

HA tagged Cdc6 was detected with 12CA5 MAb (1:100), while myc tagged Cdc6 was detected by MAb 9E10 (1:200); the signal was amplified as previously described (Piatti et al., 1995). Anti-Swi6 Ab were used at 1:100,000 and anti-Cdc28 Ab at 1:1000 dilution.

Secondary antibodies were purchased from Amersham and proteins were detected by an enhanced chemiluminescence system according to the manifacturer.

vi) Immunoprecipitation and kinase assays

Protein extracts were prepared as in Schwob et al., 1994. For Clb2 kinase assays, 100 mg of total proteins were immunoprecipitated using anti-Clb2 Ab (1:30, Amon et al., 1992). To assay Cdc6-associated kinase, HA3Cdc6 or Cdc6HA3 was immunoprecipitated with 12CA5 Ab (1:10) from the amounts of protein extracts indicated in figure legends. Histone H1 kinase activity was measured as previously described (Schwob et al., 1994).

vii) Other Techniques

Flow cytometric DNA quantitation was determined according to Epstein and Cross (1992) on a Becton-Dickinson FACScan. *In situ* immunofluorescence and photomicroscopy were performed according to Nasmyth et al. (1990). To detect immunostaining of myc12Cdc6, 9E10 MAb was used at a 1:5 dilution and the signal was detected by indirect immunofluorescence using CY3-conjugated anti-mouse Ab (1:200, Sigma).

Example 1

Pre-RCs cannot be formed in G2/M

*cdc15 GAL-ubiCDC6* (K5033) cells were arrested into either α factor or nocodazole after having been deprived of Cdc6 protein (see Materials and Methods). During the arrests the synthesis of Cdc6 protein was re-induced by shift to galactose medium. The triangles in Fig. 1 indicate increasing concentrations of DNAse I. The position of the ORC-induced hypersensitive site is indicated as an asterisk.

Example 2

Cells deprived of Cdc6 in late mitosis are unable to replicate in the next cell cycle but undergo reductional" anaphase
*cdc15* (K1993) and *cdc15 GAL-ubiCDC6* cells (K5032) growing in YEPRG medium were arrested by 3 hours incubation at 37°C. Afterwards, cells were filtered, resuspended in YEPR at 37°C and after 30′ (time=0) released into either YEPR (Fig. 2A and B) or YEPRG medium at 25°C (Fig. 2C). In Fig. 2D) are shown, for the same experiment described in Fig. 2A), B) and C), the fractions of 2C nuclei (i.e. nuclei which have undergone DNA replication) calculated with the following equation :
%2C = $100.(2.C_4 + U - C_1)/(U + 2.B)$ , where $C_1$, $C_2$, and $C_4$ are the fractions of cells at each point with 1C, 2C, or 4C DNA contents, and U and B the fractions of uni- and binucleated cells respectively (see Materials and Methods).

Example 3

Cells pass a "point of no return" at the time of S phase entry after which Cdc6 synthesis is unable to promote DNA replication

A) *cdc15* (K1993) and *cdc15 GAL-ubiCDC6* (K5032) cells were treated as in Example 2. After release from the *cdc15* arrest in YEPR (t=0), aliquots of the *cdc15 GAL-ubiCDC6* culture, withdrawn at 10′ intervals (indicated on the right in Fig. 3A), were supplemented with 2% galactose to re-induce Cdc6 synthesis. For each independent culture, cell samples were analysed by FACS every 30 minutes for 2 hours. The fractions of 2C nuclei at each time point has been calculated using the formula described in Example 2 (Fig. 2D). The time indicated on the right of Fig. 3A represents the time at which each aliquot of culture has been transferred to galactose medium.

B) The same experimental procedure used in A) has been applied to a *GAL-HA3CDC6* strain (K5763), with the exception that 0.1% galactose has been used to re-induce Cdc6 synthesis (Fig. 3B). Under the same experimental conditions as in B), expression of HA3Cdc6 at the different time points has been evaluated by Western analysis after 30′ of induction. Swi6p was used as an internal loading control (Fig. 3C).

Example 4

Deletion of *CLB5* and *CLB6* causes a delay in S phase entry and a similar delay in the "point of no return"
*cdc15* (K1993) and *cdc15 clb5 clb6* (K5027) cells growing in YEPD were arrested for 3.5 hours at 37°C and then released at 25°C. At different time points cell samples were withdrawn for FACS analysis and budding index (Fig. 4A) and to assay Clb2-dependent histone H1 kinase activity (Fig. 4B). C) *cdc15 clb5 clb6 GAL-ubiCDC6* cells (K5231) were treated as described for K5032 in Example 3. After release from *cdc15* arrest into YEPR (t=0), aliquots of K5321 culture, withdrawn at 10 minutes intervals, were supplemented with 2% galactose and incubated in YEPRG for 180 minutes. From each individual culture, cell samples were collected every 30 minutes for FACS analysis and the fractions of 2C nuclei have been evaluated using the equation as described in Example 2. The results are shown in Fig. 4C.

Example 5

B-type cyclin dependent Cdc28 kinase associates with Cdc6p *in vivo* during S, G2 and M phases.

A) Cell extracts from wild type cells containing an untagged (K699) or a HA3 tagged version of *CDC6* (*CDC6HA3*, K4528) have been incubated in either the presence (+Ab) or in the absence (-Ab) of 12CA5 Ab. After immunoprecipitation, histone H1 kinase has been assayed (Fig. 5A).

B) K4528 (wild type), K5275 (*cdc28-13*), K5082(*cdc34-2*), K5272(*cdc7-1*), K5074(*cdc34-2, sic1*), K5279(*cdc15-2*) cells, all containing the *CDC6HA3* gene, have been grown in YEPD at 25°C and arrested for 3.5 hours at 37°C. K4528 cells have also been incubated in YEPD with 80 mM hydroxyurea (HU) at 25°C for 3.5 hours. Cell samples have been collected for Western blot with the 12CA5 Ab(lower panel of Fig. 5B), kinase assays and FACS analysis (not shown). C (loading control) is a protein cross-reacting with the 12CA5 Ab. For kinase assays, Cdc6p was immunoprecipitated with 12CA5 Ab from 0.5 mg of protein extracts and histone H1 kinase activity was measured (Fig. 5B).

C) Cdc6-associated kinase is dependent on CDC28. Wild type (WT, K4527) and cdc28-4 (K5472) cells, both containing 5 copies of GAL-HA3CDC6, were grown in YEPR and induced for 4 hours in YEPRG at 25°C; K5472 cells were also arrested for 3 hours in YEPR at 37°C or incubated for 1 hour in YEPRG at 25°C and then arrested for 3 hours at 37°C. For each condition 0.2 mg of protein extracts were immunoprecipitated with 12CA5 Ab and the associated kinase activity was measured (Fig. 5C. R: Raffinose. G: Galactose).

D) Cdc6-associated kinase is inhibited by p40$^{SIC1}$. 1 mg of protein extract from K4527, grown in YEPR at 25°C or induced for 4 hours in YEPRG, was immunoprecipitated with 12CA5 Ab and before the last washing step split into four aliquots. Kinase activity was measured in the absence or in the presence of the indicated amounts of purified p40$^{SIC1}$. R:Raffinose. G: Galactose. The addition of p40$^{SIC1}$ causes the appearance of two additional signals probably due to phosphorylation of p$_{40}$$^{SIC1}$ by an aspecific kinase. E) Cdc28p co-immunoprecipitates with Cdc6p. Cdc6p was immunoprecipitated with 12CA5 Ab from protein extracts (5 mg) of K4527 cells grown at 25°C in YEPR and induced for 4 hours in YEPRG. Protein A-Sepharose beads were boiled in SDS buffer and analysed by Western blot using Cdc28 Ab (1:500) (Fig. 5D. R: Raffinose. G: Galactose).

REFERENCES

Amon, A., Surana, U., Muroff, I. and Nasmyth, K. (1992). Regulation of p34CDC28 tyrosine phosphorylation is not required for entry into mitosis in S. cerevisiae. Nature 355, 368-371.

Amon, A., Irniger, S. and Nasmyth, K. (1994). Closing the cell cycle circle in yeast: G2 cyclin proteolysis initiated at mitosis persists until activation of G1 cyclins in the next cycle. Cell 77, 1037-1050.

Bell, S.P. and Stillman, B. (1992). ATP-dependent recognition of eukaryotic origins of DNA replication by a multiprotein complex. Nature 357, 128-134.

Blow, J.J. and Laskey, R.A., (1988), Nature 332, 546-547

Broek, D., Bartlett, R., Crawford, K., and Nurse, P. (1991). Involvement of p34Cdc2 in establishing the dependency of S phase on mitosis. Nature 349, 388-393

Bueno, A. and Russell, P. (1992). Dual function of CDC6: a yeast protein required for DNA replication also inhibits nuclear division. EMBO J. 11, 2167-2176.

Choi, W., Clark, M.W., Chen, J.X. and Jong, A.Y. (1990). The CDC4 gene product is associated with the yeast nuclear skeleton. Bioch. Biophys. Res. Comm. 172, 1324-1330.

Cocker, J.H., Piatti, S., Santocanale, C., Nasmyth, K. and Diffley, J. (1996). An essential role for the Cdc6 protein in forming the pre-replicative complexes of budding yeast. Nature 379, 180-182.

Dahmann, C., Diffley, J.F.X. and Nasmyth, K. (1995). S-phase-promoting cyclin-dependent kinases prevent re-replication by inhibiting the transition of replication origins to a pre-replicative state. Curr. Biol. 5, 1257-1269.

Dalton, S. and Whitbread, L. (1995). Cell cycle-regulated nuclear import and export of Cdc47, a protein essential for initiation of DNA replication in budding yeast. Proc. Natl. Acad. Sci. USA 92, 2514-2518.

Diffley, J.F.X. and Cocker, J.H. (1992). Protein-DNA interactions at a yeast replication origin. Nature 357, 169-172.

Diffley, J.F.X., Cocker, J.H., Dowell, S. and Rowley, A. (1994). Two steps in the assembly of complexes at yeast replication origins in vivo. Cell 78, 303-316.

Dirick, L. Böhm, T. and Nasmyth, K. (1995). Roles and regulation of Cln-Cdc28 kinases at the start of the cell cycle of Saccharomyces cerevisiae. EMBO J. 14, 4803-4813.

Epstein, C.B. and Cross, F.R. (1992). CLB5: a novel B cyclin from budding yeast with a role in S phase. Genes Dev. 6, 1695-1706.

Fitch, I., Dahmann, C., Surana, U., Amon, A., Nasmyth, K., Goetsch, L., Byers, B. and Futcher, B. (1992). Chracterization of four B-type cyclin genes of the budding yeast Saccharomyces cerevisiae. Mol. Biol. Cell 3, 805-818.

Fox, C.A., Loo, S., Dillin, A. and Rine, J. (1995). The origin recognition complex has essential functions in transcriptional silencing and chromosomal replication. Genes Dev. 9, 911-924.

Harlow, E. and Lane, D., 1988, Antibodies, A Laboratory Manual, Coldspring Harbour Laboratory, 139 - 237.

Hartwell, L.H. (1976). Sequential function of gene products relative to DNA synthesis in the yeast cell cycle. J. Mol. Biol. 104, 803-817.

Hartwell, L.H. and Weinert, T.A. (1989). Checkpoints: controls that ensure the order of cell cycle events. Science 246, 629-634.

Hayles, J., Fisher, D., Woollard, A., and Nurse, P. (1994). Temporal order of S phase and mitosis in fission yeast is determined by the state of p34Cdc2 mitotic B cyclin complex. Cell. 78, 813-822.

Hennessy, K.M. and Botstein, D. (1990). Subcellular localisation of yeast CDC46 varies with the cell cycle. Genes Dev. 4, 2252-2263.

Hogan, E. and Koshland, D. (1992). Addition of extra origins of replication to a minichromosome suppresses its mitotic loss in *cdc6* and *cdc14* mutants of *Saccharomyces cerevisiae*. Proc. Natl. Aced. Sci. USA 89, 3098-3102.

Irniger, S., Piatti, S., Michaelis, C and Nasmyth, K. (1995). Genes involved in sister chromatid separation are needed for B-type cyclin proteolysis in budding yeast. Cell 81, 269-278.

Jackson, A.L., Pahl, P.M., Harrison, K., Rosamond, J. and Sclafani R.A. (1993). Cell cycle regulation of the yeast Cdc7 protein kinase by association with the Dbf4 protein. Mol. Cell. Biol. 13, 2899-2908.

Koch, C. and Nasmyth, K. (1994). Cell cycle regulated transcription in yeast. Curr. Opin. Cell Biol. 6, 451-459.

Liang, C., Weinreich, M. and Stillman B. (1995). ORC and Cdc6p interact and determine the frequency of initiation of DNA replication in the genome. Cell 81, 667-676.

Maniatis, T., Fritsch, T.F. and Sambrook, J. (1982). Molecular Cloning: A Laboratory Manual (Cold Spring Harbor, New York: Cold Spring Harbor Laboratory).

Mendenhall, M.D. (1993). An inhibitor of p34CDC28 protein kinase activity from *Saccharomyces cerevisiae*. Science 259, 216-219.

Micklem, G., Rowley, A., Harwood, J., Nasmyth, K. and Diffley, J. (1993). Yeast origin recognition complex is involved in DNA replication and transcriptional silencing. Nature 366, 87-89.

Moreno, S. and Nurse, P. (1994). Regulation of progression through the G1 phase of the cell cycle by the Rum1 gene. Nature. 367, 236-242.

Mortimer, R.K. and Hawthorne, D.C. (1969). Yeast Genetics. In the Yeasts, Volume 1, A.M. Rose and J.S. Harrison, eds (New York: Academic Press), 385-460.

Muzi-Falconi, M., Brown, G.W. and Kelly, T. (1996). *cdc18+* regulates initiation of DNA replication in *Schizosaccharomyces pombe*. Proc. Natl. Acad. Sci. USA. in press.

Nishitani, H. and Nurse, P. (1995). p65*cdc18* plays a major role controlling the initiation of DNA replication in fission yeast. Cell 83, 397-405.

Piatti, S., Lengauer, C and Nasmyth, K. (1995). Cdc6 is an unstable protein whose *de novo* synthesis in G1 is important for the onset of S phase and for preventing a "reductional" anaphase in the budding yeast *Saccharomyces cerevisiae*. EMBO J. 14, 3788-3799.

Piatti, S., Bohm, T., Cocker, J., Diffley, J.F.X., and Nasmyth, K. (1996). Activation of S phase promoting Cdks in late G1 defines a "point of no return" after which Cdc6 synthesis cannot promote DNA replication in yeast. Genes and Development in press.

Schwob, E. and Nasmyth, K. (1993). *CLB5* and *CLB6*, a new pair of B cyclins involved in S phase and mitotic spindle formation in *S. cerevisiae*. Genes Dev. 7, 1160-1175.

Schwob, E., Böhm, T., Mendenhall, M.D. and Nasmyth, K. (1994). The B-type cyclin kinase inhibitor p40*SIC1* controls the G1 to S transition in *S. cerevisiae*. Cell 79, 233-244.

Surana, U., Robitsch, H., Price, C., Schuster, T., Fitch, I., Futcher, A.B. and Nasmyth, K. (1991). The role of CDC28 and cyclins during mitosis in the budding yeast *S. cerevisiae*. Cell 65, 145-161.

Surana, U., Amon, A., Dowzer, C., Mcgrew, J., Byers, B. and Nasmyth, K. (1993). Destruction of the CDC28/CLB kinase is not required for metaphase/anaphase transition in yeast. EMBO J. 12, 1969-1978.

Tyers, M., Tokiwa, G., Nash, R. and Futcher, B. (1992). The Cln3-Cdc28 kinase complex of *S. cerevisiae* is regulated by proteolysis and phosphorylation. EMBO J. 11, 1773-1784.

Yan, H., Merchant, M. and Tye, B.K. (1993). Cell cycle-regulated nuclear localization of MCM2 and MCM3, which are required for the initiation of DNA synthesis at chromosomal replication origins in yeast. Genes Dev. 7, 2149-2160.

Yoon, H.J., Loo, S. and Campbell, J.L. (1993). Regulation of *Saccharomyces cerevisiae* CDC7 function during the cell cycle. Mol. Biol. Cell 4, 195-208.

Zhou et al., 1989; J. Biol. Chem. 264, 9022-9029.

Zwerschke, W., Rottjakob, H.W. and Küntzel, H. (1994). The *Saccharomyces cerevisiae CDC6* gene is transcribed at late mitosis and encodes a ATP/GTPase controlling S phase initiation. J. Biol. Chem. 269, 23351-23356.

## Claims

1. Compounds which interfere with the function of the cdc6 protein to form or maintain pre-replication complexes in an animal cell without impairing the cell's ability to activate cyclin dependent kinases that promote S phase and/or M phase, for use in a method of killing rapidly dividing cells, in particular tumour cells, by inhibiting DNA replication.

2. Compounds of claim 1 for use in cancer therapy.

3. A method of screening for compounds of claim 1 with the ability to interfere with DNA replication in an animal cell, wherein a test compound is applied to a substrate comprising the components required for DNA replication, and wherein the effect of the substance on the ability of the cdc6 protein to form or maintain pre-replication complexes is determined directly or indirectly.

4. The method of claim 3, wherein a substrate comprising a mixture of an egg extract, which is capable of DNA replication, and chromatin, is incubated with the test compound for a period of time sufficient for cdc6p to form pre-replication complexes, and wherein assembly of the Cdc6 protein onto chromatin is determined directly.

5. The method of claim 3, wherein a substrate comprising a mixture of human sperm chromatin or nuclei isolated from human G1 cells, an extract prepared from human cells that express high levels of cdc6p, and a cyclin dependent kinase inhibitor, is incubated with the test compound for a period of time sufficient for cdc6p to form pre-replication complexes, and wherein assembly of the Cdc6 protein onto chromatin is determined directly.

6. The method of of claim 4 or 5, wherein assembly of Cdc6 protein onto chromatin is determined by a anti-cdc6 antibody.

7. The method of claim 6, wherein the anti-cdc6 antibody carries an fluorescent label.

8. The method of claim 3, wherein the substrate comprises rapidly dividing animal cells, in particular human cells derived from a tumour cell line, wherein the cells are incubated in the presence of the test substance for a period of time sufficient for cdc6p to form pre-replication complexes, and wherein the cells are lysed and assembly of Cdc6 protein onto chromatin is determined directly.

9. The method of claim 3, wherein the substrate comprises yeast cells that carry an orc mutation and an inducible plasmid containing the human CDC6 sequence such that they overexpress cdc6p, wherein the cells are grown and incubated with the test substance, and wherein the effect of the compound to inhibit cdc6p function is determined indirectly by determining survival of the cells.

10. A method of any one of claims 3 to 9, wherein the ability of the compound not to impair activation of cyclin dependent kinases that promote S phase and/or M phase is determined by measuring kinase activity and/or cyclin accumulation.

11. Pharmaceutical composition comprising as an active ingredient a compound as defined in claim 1.

## Fig. 1

Fig. 2

**A** cdc15 WT-CDC6

**B** cdc15 GAL-ubiCDC6 (off)

**C** cdc15 GAL-ubiCDC6 (off→on)

**D**

## Fig. 3A

**A**

### cdc15

### cdc15   GAL-ubiCDC6
### (off)

### cdc15   GAL-ubiCDC6
### (off→on)

t=0

t=10'

t=20'

t=30'

t=40'

t=70'

**Fig. 3B**

**Fig. 3C**

C

←Swi6p

←Cdc6p

Fig. 4A

**Fig. 4B**

**Fig. 4C-1**

C

cdc15

cdc15 clb5 clb6

cdc15 clb5 clb6
GAL-ubi CDC6
(off)

cdc15 clb5 clb6
GAL-ubi CDC6
(off→on)

**Fig. 4C-2**

Fig. 5A

A        WT

histone H1
kinase

B

——G1—— S —— G2—— M——
cdc28   cdc7   cdc34sic1  cdc15
cdc34   HU

histone H1
kinase

← Cdc6p
← C

Fig. 5B

C

wt *cdc28-4*

25°C 25°C 37°C

R | G | R | G | R | G

← histone H1 kinase

D

p40$^{SIC1}$ - -  20ng 2ng

R | G | | R | G | R | G

← histone H1 kinase

E

R | G

← Cdc28p

## Fig. 6

POINT OF NO RETURN

**European Patent** **PARTIAL EUROPEAN SEARCH REPORT** Application Number

**Office** which under Rule 45 of the European Patent Convention EP 96 10 9483
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | CELL,<br>vol. 78, 1994,<br>pages 303-316, XP002020143<br>J.F.X. DIFFLEY ET AL.: "Two steps in the assembly of complexes at yeast replication origins in vivo."<br>* page 309, right-hand column - page 311, right-hand column *<br>--- | 1-11 | A61K49/00<br>A61K31/00 |
| X | EMBO J., VOL. 14, NO. 15, PAGE(S) 3788-99, 1995, XP002020144<br>PIATTI, SIMONETTA ET AL: "Cdc6 is an unstable protein whose de novo synthesis in G1 is important for the onset of S phase and for preventing a 'reductional' anaphase in the budding yeast Saccharomyces cerevisiae"<br>* abstract *<br>* page 3792 *<br>* figure 6 *<br>--- | 1-11 | |
| | -/-- | | **TECHNICAL FIELDS SEARCHED (Int.Cl.6)**<br><br>A61K |

### INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 3 December 1996 | Dullaart, A |

## PARTIAL EUROPEAN SEARCH REPORT

European Patent Office

Application Number

EP 96 10 9483

### DOCUMENTS CONSIDERED TO BE RELEVANT

| Category | Citation of document with indication, where appropriate, of relevant passages | Relevant to claim |
|---|---|---|
| O,X | 28TH ANNUAL MEETING OF THE SWISS SOCIETIES FOR EXPERIMENTAL BIOLOGY (USGEB/USSBE), ZUERICH-IRCHEL, SWITZERLAND, MARCH 27-29, 1996., XP002020145 PIATTI S ET AL: "Activation of S phase promoting CDKs in yeast defines a "point of no return" after which CDC6 can no longer promote DNA replication." & EXPERIENTIA (BASEL), VOL. 52, NO. ABSTR., PUBL. 1996, PAGE(S) A11, ABSTR. NO. S05-02, * abstract * --- | 1-11 |
| D,X | CELL, vol. 79, 21 October 1994, pages 233-244, XP002020146 E. SCHWOB ET AL.: "The B-type cyclin kinase inhibitor p40SIC1 controls the G1 to S transition in S. Cerevisiae." * abstract * * page 233, right-hand column * * page 234, right-hand column - page 235 * --- | 1-11 |
| X | BIOCHEMISTRY, VOL. 34, NO. 13, PAGE(S) 4402-11, 1995, XP002020147 KAST, CHRISTINA ET AL: "Membrane Topology of P-Glycoprotein As Determined by Epitope Insertion: Transmembrane Organization of the N-Terminal Domain of mdr3" * abstract * * page 4404, right-hand column * * page 4406, right-hand column - page 4407 * --- -/-- | 3-10 |

CLASSIFICATION OF THE APPLICATION (Int.Cl.6)

TECHNICAL FIELDS SEARCHED (Int.Cl.6)

EPO FORM 1503 03.82 (P04C10)

European Patent Office

**PARTIAL EUROPEAN SEARCH REPORT**

Application Number

EP 96 10 9483

## DOCUMENTS CONSIDERED TO BE RELEVANT

| Category | Citation of document with indication, where appropriate, of relevant passages | Relevant to claim |
|---|---|---|
| X | DATABASE INTERNET http://www.icnet.uk/lis/ipub/scirep95, "SCIENTIFIC REPORT 1995 of the Imperial Cancer Research Fund for the year 1st August 1994 to July 1995" XP002020153 * page 2 * | 1-11 |
| X | & DATABASE INTERNET http://www.icnet.uk/lis/ipub/scirep95/041.html, 1995 J.F.X. DIFFLEY ET AL.: "the Scientific Report 1995 of the Chromosome Replication Laboratory" * the whole document * | 1-11 |
| X | CELL, VOL. 81, NO. 5, PAGE(S) 667-76, 1995, XP002020148 LIANG, CHUN ET AL: "ORC and Cdc6p interact and determine the frequency of initiation of DNA replication in the genome" * abstract * * Discussion * * figures * | 1-11 |
| X | NATURE (LONDON), VOL. 379, NO. 6561, PAGE(S) 180-182., 11 January 1996, XP002020149 COCKER J H ET AL: "An essential role for the Cdc6 protein in forming the pre-replicative complexes of budding yeast." * the whole document * | 1-11 |

-/--

**CLASSIFICATION OF THE APPLICATION (Int.Cl.6)**

**TECHNICAL FIELDS SEARCHED** (Int.Cl.6)

EPO FORM 1503 03.82 (P04C10)

**European Patent Office**

## PARTIAL EUROPEAN SEARCH REPORT

Application Number

EP 96 10 9483

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| X | J. BIOL. CHEM., VOL. 269, NO. 52, PAGE(S) 33171-8, 1994, XP002020150 HANDLEY-GEARHART, PATRICIA M. ET AL: "Human ubiquitin-activating enzyme, E1. Indication of potential nuclear and cytoplasmic subpopulations using epitope-tagged cDNA constructs" * abstract * * Experimental procedures * --- | 3-10 | |
| X | J. BIOL. CHEM., VOL. 267, NO. 24, PAGE(S) 16883-8, 1992, XP002020151 WADZINSKI, BRIAN E. ET AL: "Amino-terminal modification of the phosphatase 2A catalytic subunit allows functional expression in mammalian cells" * abstract * * Experimental procedures * --- | 3-10 | **TECHNICAL FIELDS SEARCHED** (Int.Cl.6) |
| X | EMBO J., VOL. 11, NO. 6, PAGE(S) 2167-76, 1992, XP002020152 BUENO, AVELINO ET AL: "Dual functions of CDC6: a yeast protein required for DNA replication also inhibits nuclear division" * the whole document * --- | 1-11 | |
| T | GENES DEV., VOL. 10, NO. 12, PAGE(S) 1516-1531, 15 June 1996, XP000609161 PIATTI, SIMONETTA ET AL: "Activation of S-phase-promoting CDKs in late G1 defines a "point of no return" after which Cdc6 synthesis cannot promote DNA replication in yeast" * page 1517, right-hand column * ----- | 1-11 | |

EPO FORM 1503 03.82 (P04C10)

European Patent Office

---

**INCOMPLETE SEARCH**

The Search Division considers that the present European patent application does not comply with the provisions of the European Patent Convention to such an extend that is not possible to carry out a meaningfull search in the state of the art on the basis of some of the claims.

Claims searched completely:
Claims searched incompletely:     1-11
Claims not searched:

Reason for the limitation of the search:

In view of the large number of compounds, which are defined by the general definitions used in the claims, the search had to be restricted for economic reasons. The search was limited to the compounds for which pharmacological data was given and/or the compounds mentioned in the claims, and to the general idea underlying the application (see Guidelines, part B, chapter III, paragraph 3.6). Moreover, a compound cannot be defined by its desired properties.

EPO Form Supplementary Sheet C (1996)